# EUROPEAN PATENT APPLICATION

(11) **EP 4 624 446 A1**
(43) Date of publication of application: **01.10.2025**
(21) Application number: 23894426.8
(22) Date of filing: 08.11.2023
(51) Int. Cl.: C07C 17/386, C07C 19/10, C07C 21/18

(54) **METHOD FOR SEPARATING HEXAFLUOROPROPYLENE FROM CHLORODIFLUOROMETHANE**

(30) Priority: 25.11.2022 JP 2022188720
(71) Applicant: AGC INC., Chiyoda-ku, Tokyo 1008405 (JP)
(72) Inventor: MITAKE, Akihito, Tokyo 100-8405 (JP)
(74) Representative: Müller-Boré & Partner Patentanwälte PartG mbB
(86) International application number: PCT/JP2023/040246
(87) International publication number: WO 2024/111416

(57) **Abstract**

A method for separating R22 and HFP includes a mixing step of obtaining a mixture for extraction which is a mixture of a first mixture including R22 and HFP and an extraction solvent including at least one chlorine-containing compound selected from the group consisting of methylene chloride, chloroform, 1,1,1-trichloroethane, 1,1,2-trichloroethane, 1,1,1,2-tetrachloroethane, 1,1,2,2-tetrachloroethane, pentachloroethane, hexachloroethane, trichloroethylene, and tetrachloroethylene, and an extraction distillation step of distilling the mixture for extraction to respectively obtain a first distillate including HFP as a main component, and a first bottom product including the extraction solvent as a main component and including R22.

## Description

### Technical Field

The present disclosure relates to a method for separating chlorodifluoromethane and hexafluoropropylene.

### Background Art

Hexafluoropropylene is a compound used as a raw material of a fluororesin or the like, and is obtained, for example, by a thermal decomposition reaction of chlorodifluoromethane. Hereinafter, hexafluoropropylene is also referred to as "HFP", and chlorodifluoromethane is also referred to as "R22".

In order to obtain high purity HFP by thermal decomposition reaction of R22, separation of unreacted R22 and generated HFP is required. Examples of a method for separating a raw material and a reaction product include distillation using a boiling point difference, for example, but since R22 and HFP form an azeotropic composition or a quasi-azeotropic composition, separation by distillation is difficult.

As a method for separating R22 and HFP, Patent Literature 1 discloses extraction distillation using a polar organic solvent such as methanol.

### Citation List

### Patent Literature

Patent Literature 1: Japanese Patent Publication (JP-B) No. S39-19624

### SUMMARY OF INVENTION

### Technical Problem

However, all of the polar organic solvents disclosed in Patent Literature 1 is a compound having a low flash point as described later. Therefore, efficient separation of R22 and HFP without using a compound having a low flash point is required.

An object of one aspect of the present disclosure is to provide a separation method for separating R22 and HFP with high efficiency from a mixture of R22 and HFP, which is difficult to separate due to formation of an azeotropic composition or a quasi-azeotropic composition, using an extraction solvent, which is a compound having a high flash point or no flash point.

### Solution to Problem

The present disclosure includes the following aspects.
<1> A method for separating chlorodifluoromethane and hexafluoropropylene, the method including:
   a mixing step of obtaining a mixture for extraction, which is a mixture of a first mixture including chlorodifluoromethane and hexafluoropropylene and an extraction solvent including at least one chlorine-containing compound selected from the group consisting of methylene chloride, chloroform, 1,1,1-trichloroethane, 1,1,2-trichloroethane, 1,1,1,2-tetrachloroethane, 1,1,2,2-tetrachloroethane, pentachloroethane, hexachloroethane, trichloroethylene, and tetrachloroethylene; and
   an extraction distillation step of distilling the mixture for extraction to respectively obtain a first distillate including hexafluoropropylene as a main component, and a first bottom product including the extraction solvent as a main component and including chlorodifluoromethane.
<2> The method for separating chlorodifluoromethane and hexafluoropropylene according to <1>, in which the chlorine-containing compound has a boiling point in a range of from 40°C to 130°C.
<3> The method for separating chlorodifluoromethane and hexafluoropropylene according to <1> or <2>, in which the chlorine-containing compound is a compound that reduces a relative volatility Rv of chlorodifluoromethane to hexafluoropropylene to less than 0.9 when the chlorine-containing compound is added in an amount equal to three times a total molar amount of the chlorodifluoromethane and the hexafluoropropylene.
<4> The method for separating chlorodifluoromethane and hexafluoropropylene according to any one of <1> to <3>, in which
   the chlorine-containing compound is a compound that reduces a relative volatility Rv of chlorodifluoromethane to hexafluoropropylene to less than 0.9 when the chlorine-containing compound is added in an amount equal to a total molar amount of the chlorodifluoromethane and the hexafluoropropylene.
<5> The method for separating chlorodifluoromethane and hexafluoropropylene according to any one of <1> to <4>, in which the extraction solvent includes at least one chlorine-containing compound selected from the group consisting of 1,1,1-trichloroethane, trichloroethylene, tetrachloroethylene, chloroform, and methylene chloride.
<6> The method for separating chlorodifluoromethane and hexafluoropropylene according to any one of <1> to <5>, in which
   a molar ratio of the chlorine-containing compound added in the mixing step is from 1/1 to 30/1 relative to a total molar amount of the chlorodifluoromethane and the hexafluoropropylene.
<7> The method for separating chlorodifluoromethane and hexafluoropropylene according to any one of <1> to <6>, further including:
   a second distillation step of distilling the first bottom product to obtain a second distillate including the chlorodifluoromethane as a main component.
<8> The method for separating chlorodifluoromethane and hexafluoropropylene according to any one of <1> to <7>, in which:
   the first mixture further includes chlorotrifluoroethylene, and
   the first bottom product further includes chlorotrifluoroethylene.
<9> The method for separating chlorodifluoromethane and hexafluoropropylene according to <7>, in which:
   the first mixture further includes chlorotrifluoroethylene,
   the first bottom product further includes chlorotrifluoroethylene, and
   the second distillate further includes chlorotrifluoroethylene.

### Advantageous Effects of Invention

According to one aspect of the present disclosure, a separation method for separating R22 and HFP with high efficiency from a mixture of R22 and HFP, which is difficult to separate due to formation of an azeotropic composition or a quasi-azeotropic composition, using an extraction solvent, which is a compound having a high flash point or no flash point.

### BRIEF DESCRIPTION OF DRAWINGS

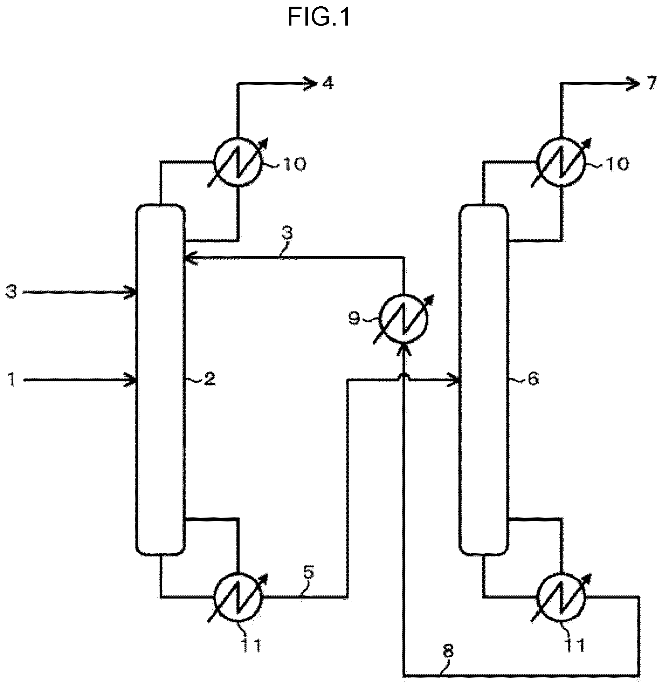

Fig. 1 is a diagram illustrating an example of a flow of a substance in a separation method of the present disclosure.

### DESCRIPTION OF EMBODIMENTS

Hereinafter, an embodiment of the present disclosure will be described in detail. Note that, the present disclosure is not limited to the following embodiment. In the following embodiment, components (including element steps and the like) are not necessarily required unless otherwise specified. The same applies to numerical values and ranges thereof, and the present disclosure is not limited thereto.

In the present disclosure, the term "step" includes not only a step independent of other steps but also a step that cannot be clearly distinguished from other steps as long as a purpose of the step is achieved.

In the present disclosure, a numerical range indicated using "to" includes numerical values described before and after "to" as a minimum value and a maximum value, respectively.

In the present disclosure, each component may contain plural corresponding substances. In a case in which plural types of substances corresponding to each component is present in a composition, a ratio of each component means a total ratio of the plural of types present in the composition unless otherwise specified.

In a case in which the embodiment is described with reference to the drawings in the present disclosure, the configuration of the embodiment is not limited to the configuration illustrated in the drawings. Sizes of members in each drawing are conceptual, and a relative relationship between the sizes of the members is not limited thereto.

In the present disclosure, a "distillate" refers to a substance distilled from a column top side of a distillation column, and a "bottom product" refers to a substance distilled from a column bottom side of the distillation column.

In the present disclosure, a "main component" means a content, amounts of components other than which are relatively small. The amount of the "main component" is preferably 50 mol% or more, more preferably 60 mol% or more, still more preferably 70 mol% or more, and most preferably 80 mol% or more relative to a total amount.

In the present disclosure, unless otherwise specified, a boiling point of a compound is a value at a normal pressure, and the normal pressure is 1.013×10⁵ Pa.

### [Separation Method]

A separation method in one embodiment of the present disclosure includes a mixing step of obtaining a mixture for extraction that is a mixture of a first mixture containing chlorodifluoromethane (R22) and hexafluoropropylene (HFP), and an extraction solvent containing at least one chlorine-containing compound selected from the group consisting of methylene chloride, chloroform, 1,1,1-trichloroethane, 1,1,2-trichloroethane, 1,1,1,2-tetrachloroethane, 1,1,2,2-tetrachloroethane, pentachloroethane, hexachloroethane, trichloroethylene, and tetrachloroethylene; and an extraction distillation step of distilling the mixture for extraction to respectively obtain a first distillate containing HFP as a main component and a first bottom product containing the extraction solvent as a main component and containing R22. Hereinafter, at least one chlorine-containing compound selected from the group consisting of methylene chloride, chloroform, 1,1,1-trichloroethane, 1,1,2-trichloroethane, 1,1,1,2-tetrachloroethane, 1,1,2,2-tetrachloroethane, pentachloroethane, hexachloroethane, trichloroethylene, and tetrachloroethylene is also referred to as a "CL compound".

The separation method of the present embodiment may include steps other than the mixing step and the extraction distillation step.

Examples of other steps include a second distillation step in which a first bottom product is distilled to obtain a second distillate containing R22 as a main component. The separation method of the present embodiment preferably further includes the second distillation step.

In the separation method of the present embodiment, highly efficient separation of R22 and HFP was achieved by using the CL compound, among compounds having a high flash point or having no flash point, as an extraction solvent.

Values of flash points of some of the CL compounds used as the extraction solvent in the separation method of the present embodiment are illustrated in Table 1 below. As a comparison, values of flash points of methanol, dimethylformamide, and acetone, which are examples of a compound having a low flash point, are also illustrated in Table 1 below. Note that, the values of flash points illustrated in the following Table 1 are values obtained by a measurement method in accordance with JIS K2265 (2007).

The CL compound used as the extraction solvent in the separation method of the present embodiment is preferably a compound having no flash point or having a flash point at 250°C or higher, more preferably a compound having no flash point or having a flash point at 300°C or higher, still more preferably a compound having no flash point or having a flash point at 350°C or higher, especially preferably a compound having no flash point or having a flash point at 400°C or higher, and extremely preferably a compound having no flash point.

**[Table 1]**

| Classification | Compound name | Flash point |
|---|---|---|
| CL compound | Chloroform | None |
| | Methylene chloride | None |
| | 1,1, 1-trichloroethane | None |
| | Trichloroethylene | None |
| | Tetrachloroethylene | None |
| Other Compounds | Methanol | 12°C |
| | Dimethylformamide | 58°C |
| | Acetone | -18°C |

Hereinafter, each step included in the separation method of the present embodiment will be described.

### <Mixing Step>

In the mixing step, the mixture for extraction that is the mixture of the first mixture containing R22 and HFP and the extraction solvent containing the CL compound is obtained.

### (First Mixture)

The first mixture contains at least R22 and HFP, and may further contain other compounds. Examples of other compounds contained in the first mixture include a compound generated by thermal decomposition reaction of R22 and the like. Specific examples of other compounds contained in the first mixture include chlorotrifluoroethylene, tetrafluoroethylene, trifluoroethylene, perfluorocyclobutane, 1,1,1,2,3,3,3-heptafluoropropane, 1,1,1,2-tetrafluoroethane, 1,1,1,2,2-pentafluoroethane, dichlorodifluoromethane and the like. Hereinafter, chlorotrifluoroethylene is also referred to as "CTFE", and tetrafluoroethylene is also referred to as "TFE".

In a case in which the first mixture contains other compounds, other compounds contained in the first mixture may include only one type or two or more types.

A total content of R22 and HFP relative to an entire first mixture is, for example, 50 mol% or more, and may be 80 mol or more, 90 mol or more, 99 mol% or more, or 100 mol%.

In a case in which the first mixture contains CTFE among other compounds, a content of CTFE relative to the entire first mixture may be less than 10 mol%, may be from 0.01 mol% to 5 mol%, or may be from 0.01 mol% to 2 mol%.

A molar ratio of HFP and R22 contained in the first mixture is not particularly limited. The molar ratio of a content of HFP to a content of R22 contained in the first mixture may be from 1/99 to 50/50, from 3/97 to 40/60, or from 5/95 to 30/70. Hereinafter, the molar ratio of the content of HFP to the content of R22 contained in the first mixture is also referred to as a "molar ratio (HFP/R22)".

In particular, a mixture of HFP and R22 having the molar ratio (HFP/R22) of 10/90 is an azeotropic composition, and separation by distillation is difficult, but according to the separation method of the present embodiment, R22 and HFP can be separated with high efficiency.

### (Extraction Solvent)

The extraction solvent contains at least the CL compound, and may further contain other compounds.

A content of the CL compound relative to an entire extraction solvent is preferably 90 mass% or more, more preferably 95 mass% or more, still more preferably 99 mass% or more, and may be 100 mass% from the viewpoint of separating R22 and HFP with high efficiency.

The CL compound contained in the extraction solvent is preferably a CL compound having from 1 to 2 carbon atoms from the viewpoint of a suitable boiling point, and more preferably a compound having 1 carbon atom from the viewpoint of low toxicity.

The number of chlorine atoms contained in one molecule of the CL compound contained in the extraction solvent is, for example, from one to six, and is preferably from one to four, and more preferably from one to three from the viewpoint of low toxicity.

The CL compound contained in the extraction solvent may be a saturated compound or an unsaturated compound in a case in which the number of carbon atoms is two or larger.

Specific examples of the CL compound include methylene chloride, chloroform, 1,1,1-trichloroethane, 1,1,2-trichloroethane, 1,1,1,2-tetrachloroethane, 1,1,2,2-tetrachloroethane, pentachloroethane, hexachloroethane, trichloroethylene, tetrachloroethylene and the like.

Among them, from the viewpoint of separating R22 and HFP with high efficiency, the examples of the CL compound preferably include at least one selected from the group consisting of 1,1,1-trichloroethane, trichloroethylene, tetrachloroethylene, chloroform, and methylene chloride, more preferably includes at least one selected from the group consisting of chloroform and methylene chloride, and still more preferably includes chloroform.

The extraction solvent may contain only one type or two or more types of the CL compound.

From the viewpoint of separating R22 and HFP with high efficiency, the CL compound preferably has a boiling point in a range of from 40°C to 130°C, more preferably has a boiling point in a range of from 40°C to 80°C, and still more preferably has a boiling point in a range of from 40°C to 65°C. In the case in which the boiling point of the CL compound is equal to or higher than the lower limit value, the boiling point differences between the CL compound and each of R22 and HFP increase, so that R22 and HFP are easily separated with high efficiency, and R22 and the CL compound are easily separated in the second distillation step and the like to be described later. In the case in which the boiling point of the CL compound is equal to or lower than the upper limit value, distillation at low temperature can be performed in the extraction distillation step and the second distillation step.

Values of the boiling points of some of the CL compounds used as the extraction solvent in the separation method of the present embodiment are illustrated in Table 2 below.

**[Table 2]**

| Compound name | Boiling point (°C) |
|---|---|
| Methylene chloride | 40 |
| Chloroform | 61 |
| 1,1, 1-trichloroethane | 74 |
| Trichloroethylene | 87 |
| Tetrachloroethylene | 121 |

The CL compound is preferably a compound which reduces a relative volatility Rv of R22 to HFP to less than 0.9 when the CL compound is added in an amount equal to three times a total molar amount of the R22 and the HFP from the viewpoint of separating R22 and HFP with high efficiency.

That is, the value of the relative volatility Rv when the CL compound is added in an amount equal to three times the total molar amount of the R22 and the HFP is preferably less than 0.9. The value of the relative volatility Rv when the CL compound is added in an amount equal to three times the total molar amount of the R22 and the HFP is preferably 0.8 or less, more preferably 0.7 or less, still more preferably 0.6 or less, and particularly preferably 0.5 or less, from the viewpoint of separating R22 and HFP with high efficiency.

The CL compound is preferably a compound which reduces the relative volatility Rv of R22 to HFP to less than 0.9 when the CL compound is added in an amount equal to the total molar amount of the R22 and the HFP from the viewpoint of separating R22 and HFP with higher efficiency.

That is, the value of the relative volatility Rv is preferably less than 0.9 also when the CL compound is added in an amount equal to the total molar amount of the R22 and the HFP. The value of the relative volatility Rv when the CL compound is added in an amount equal to the total molar amount of the R22 and the HFP is preferably 0.8 or less, more preferably 0.7 or less, still more preferably 0.6 or less, and particularly preferably 0.5 or less, from the viewpoint of separating R22 and HFP with higher efficiency.

For example, since the mixture composed of R22 and HFP having the molar ratio (HFP/R22) of about 10/90 forms an azeotropic composition, the relative volatility Rv is close to 1, and separation by normal distillation is difficult. In contrast, by adding the CL compound to the mixture of R22 and HFP, the relative volatility Rv becomes a value away from 1, and separation becomes easy. It is presumed that this is because the boiling point of the CL compound is higher than the boiling points of R22 and HFP, and affinity between the CL compound and R22 is high, so that R22 is less likely to volatilize, and the affinity between the CL compound and HFP is low, so that volatilization of HFP is less likely to be hindered.

Therefore, in the mixing step, from the viewpoint of separating R22 and HFP with high efficiency, it is preferable to select the type of the CL compound and adjust an added amount of the CL compound so that the relative volatility Rv in the mixture for extraction is less than 0.9. In the mixing step, it is more preferable to select the type of the CL compound and adjust the added amount of the CL compound so that the relative volatility Rv is 0.8 or less, it is further preferable to select the type of the CL compound and adjust the added amount of the CL compound so that the relative volatility Rv is 0.7 or less, it is particularly preferable to select the type of the CL compound and adjust the added amount of the CL compound so that the relative volatility Rv is 0.6 or less, it is extremely preferable to select the type of the CL compound and adjust the added amount of the CL compound so that the relative volatility Rv is 0.5 or less, and it is most preferable to select the type of the CL compound and adjust the added amount of the CL compound so that the relative volatility Rv is 0.4 or less.

The relative volatility Rv of R22 to HFP is represented by the following Formula (3). Rv = (molar fraction of R22 in gas phase part/molar fraction of R22 in liquid phase part)/(molar fraction of HFP in gas phase part/molar fraction of HFP in liquid phase part)

The relative volatility Rv of R22 to HFP is measured as follows.

Specifically, the mixture of HFP and R22 having the molar ratio (HFP/R22) of 10/90 and the extraction solvent as necessary are injected into a 1-liter autoclave equipped with a pressure gauge, temperature is adjusted so that a gauge pressure of 0.19 MPaG is obtained, and the autoclave is held for one day to stabilize the composition in the autoclave. Note that, in a case in which the value of the relative volatility Rv when the CL compound is added in an amount equal to three times the total molar amount of the R22 and the HFP is measured, the CL compound is added in an amount equal to three times the total molar amount of the R22 and the HFP as the extraction solvent to be injected as necessary.

Next, a measurement sample is collected from each of the gas phase and the liquid phase, completely gasified, and then analyzed by gas chromatography to calculate the relative volatility Rv of R22 to HFP.

That is, the relative volatility Rv means a value when the molar ratio (HFP/R22) is 10/90 and the gauge pressure is 0.19 MPaG.

The molar ratio of the added amount of the CL compound in the mixing step is preferably from 1/1 to 30/1, more preferably from 1/1 to 15/1, and still more preferably from 3/1 to 10/1 relative to the total molar amount of the R22 and the HFP from the viewpoint of separating R22 and HFP with high efficiency. Hereinafter, the molar ratio of the added amount of the CL compound to the total molar amount of the R22 and the HFP in the mixing step is also referred to as a "molar ratio (CL/(R22+HFP))".

For example, as described later, in a case in which the first mixture containing R22 and HFP is supplied to an extraction distillation column and the extraction solvent containing the CL compound is further supplied to the extraction distillation column, the "molar ratio (CL/(R22+ HFP))" represents a molar ratio of a supply amount of the CL compound supplied to the extraction distillation column to a total supply amount of the R22 and the HFP supplied to the extraction distillation column.

A timing of adding the extraction solvent to the first mixture in the mixing step is not particularly limited as long as this is before the extraction distillation step. Note that, from the viewpoint of efficiency of a distillation operation, it is preferable to perform the extraction distillation step simultaneously with the mixing step in which, after the first mixture is supplied to the extraction distillation column, the extraction solvent is further supplied to the extraction distillation column to prepare the mixture for extraction in the column.

Note that, in the mixing step, for example, a mixture of the first mixture and the extraction solvent is obtained by adding the extraction solvent to the first mixture, but an operation of mixing the first mixture and the extraction solvent may be separately performed.

### <Extraction Distillation Step>

In the extraction distillation step, the mixture for extraction obtained in the mixing step is distilled to respectively obtain a first distillate containing HFP as a main component and a first bottom product containing the extraction solvent as a main component and containing R22.

The extraction distillation step can be performed using a commonly used distillation apparatus, for example, a distillation column such as a plate column, a packed column and the like. Various conditions of the extraction distillation step, for example, operation temperature, an operation pressure, a reflux ratio, the total number of stages of the distillation column, a position of a preparation stage, a position of an extraction solvent supply stage and the like are not particularly limited, and can be selected as appropriate in order to achieve an intended separation. In a case in which the distillation column as the plate column is used in the extraction distillation step, the number of stages of the distillation column is, for example, from 1 to 100, and is preferably 30 or more, and more preferably 50 or more from the viewpoint of obtaining high-purity HFP. Since both R22 and HFP have low boiling points, it is preferable to perform extraction distillation under pressure, and for example, it is preferable to set the pressure to from 0 to 5 MPaG (gauge pressure).

Furthermore, temperature at a column top and a column bottom of the distillation column depends on the operating pressure and a composition of a distillate and a bottom product. In consideration of the temperatures of a condenser and a re-heater provided at the column top and the column bottom, in order to economically perform the distillation operation, the temperature at the column top is preferably set to from -60°C to 100°C, and the temperature at the column bottom is preferably set to from 20°C to 300°C. The extraction distillation may be a batch type or a continuous type, and may be a semi-continuous type in which distillates and bottom products are intermittently extracted or intermittently charged according to circumstances; however, the extraction solvent is preferably continuously supplied to the distillation apparatus.

The extraction solvent has affinity for R22. Therefore, by extracting and distilling the mixture for extraction containing R22, HFP, and the extraction solvent, the first distillate containing HFP as the main component is obtained from a column top side of the extraction distillation column. A composition of the first distillate is not limited as long as the first distillate contains HFP as the main component, but a molar fraction of HFP in the first distillate is preferably 90 mol% or more, more preferably 99 mol% or more, still more preferably 99.9 mol% or more, and may be 100 mol%. A molar fraction of R22 in the first distillate is preferably 1/100 or less, and more preferably 1/500 or less, relative to the molar fraction of R22 in the first mixture, and may be 0. The molar fraction of R22 in the first distillate is preferably 10 mol% or less, more preferably 1 mol% or less, further preferably 0.1 mol% or less, and may be 0 mol%.

In a case in which the first distillate contains compounds other than HFP and CTFE to be described later, the separation method of the present embodiment may further include a first removal step of removing the other compounds from the first distillate as necessary.

From a column bottom side of the extraction distillation column, the first bottom product containing the extraction solvent as the main component and containing R22 having affinity for the extraction solvent is obtained. The molar fraction of R22 to the sum of R22 and HFP contained in the first bottom product is preferably 70 mol% or more, more preferably 80 mol% or more, still more preferably 90 mol% or more, particularly preferably 98 mol% or more, and may be 100 mol%. The molar fraction of HFP to the sum of R22 and HFP contained in the first bottom product is preferably 1/4 or less, more preferably 1/10 or less, and still more preferably 1/20 or less, relative to the molar fraction of HFP in the first mixture, and may be 0. The molar fraction of HFP to the sum of R22 and HFP contained in the first bottom product is preferably 30 mol% or less, more preferably 20 mol% or less, still more preferably 10 mol% or less, particularly preferably 2 mol% or less, and may be 0 mol%.

Note that, in a case in which the first mixture contains CTFE, the first bottom product contains CTFE.

The first bottom product preferably further undergoes the second distillation step to be described below.

### <Second Distillation Step>

In the second distillation step, the first bottom product is distilled to obtain a second distillate containing R22 as a main component. At that time, a second bottom product containing the extraction solvent as a main component is obtained. Since a difference in boiling point between the extraction solvent contained in the first bottom product and R22 as an affinity component thereof is large, the second distillation step can be easily performed by a normal distillation separation operation. The second distillation step can be performed using a distillation apparatus similar to that in the extraction distillation step described above. Various conditions of the second distillation step, for example, operation temperature, an operation pressure, a reflux ratio, the total number of stages of the distillation column, a position of a preparation stage and the like are not particularly limited, and can be selected as appropriate in order to achieve an intended separation.

In the second distillation step, the extraction solvent and R22 are separated, and the second distillate including R22 is obtained from the column top side of the distillation column, in which a molar fraction of R22 relative to the sum of R22 and HFP is higher than the molar fraction of R22 relative to the sum of R22 and HFP in the first mixture, i.e., R22 is concentrated compared to the first mixture. The molar fraction of R22 in the second distillate is preferably 70 mol% or more, more preferably 80 mol% or more, further preferably 98 mol% or more, and may be 100 mol%. The second distillate containing a high concentration of R22 may be reused as a raw material for producing HFP.

Note that, in a case in which the first mixture contains CTFE, the second distillate also contains CTFE. In a case in which the second distillate contains CTFE, an adsorption step may further be included, in which the second distillate is brought into contact with an adsorbent to obtain a purified product containing R22 as a main component. In a case in which the second distillate contains compounds other than R22 and CTFE, the separation method of the present embodiment may further include a second removal step of removing the other compounds from the second distillate as necessary.

In the second distillation step, the second bottom product containing an extremely high concentration of the extraction solvent is obtained from the column bottom side of the distillation column. The obtained second bottom product can be directly supplied to the extraction distillation step and reused as the extraction solvent. It is possible to further purify the second bottom product to recover the extraction solvent and reuse the same in the extraction distillation step.

In the present embodiment, as described above, R22 and HFP contained in the first mixture are efficiently separated. As a result, HFP is obtained in a high concentration as the main component of the first distillate in the extraction distillation step, and R22 that is concentrated relative to the first mixture is obtained as the second distillate in the second distillation step.

Next, a flow of a substance in the separation method of the present embodiment will be described with reference to Fig. 1.

As illustrated in Fig. 1, a first mixture 1 containing R22 and HFP in a predetermined ratio, for example, a molar ratio (HFP/R22) of 10/90 is supplied to an extraction distillation column 2 operated by, for example, pressurization. As the extraction distillation column 2, one including 1 to 100 stages is used, and the first mixture 1 is supplied to the extraction distillation column 2. Then, an extraction solvent 3 containing a CL compound in a molar fraction of 1 to 30 times the total molar fraction of R22 and HFP contained in the first mixture 1 is supplied to a stage above a supply stage of the first mixture 1 in the extraction distillation column 2. Distillation is performed in this manner, and a first distillate 4 containing HFP, which is a component having no affinity for the extraction solvent 3, as a main component is extracted from a column top side of the extraction distillation column 2. In the resulting first distillate 4, the molar fraction of HFP relative to the sum of R22 and HFP is increased compared to a similar molar fraction in the first mixture 1. As a result, a mixture is obtained as the first distillate 4, in which the molar fraction of HFP is increased relative to that in the first mixture 1, indicating that HFP is concentrated.

A mixture containing the extraction solvent as a main component and containing R22 is extracted as a first bottom product 5 from a column bottom side of the extraction distillation column 2. Next, the first bottom product 5 is supplied into a solvent recovery column 6, which is another distillation column operated by, for example, pressurization, to obtain a second distillate 7 substantially free of extraction solvent from the column top side. In the resulting second distillate 7, the molar fraction of R22 relative to the sum of R22 and HFP is increased compared to a similar molar fraction in the first mixture 1. As a result, a mixture is obtained as the second distillate 7, in which the molar fraction of R22 is increased relative to that in the first mixture 1, indicating that R22 is concentrated.

Note that, in the present specification, the term "substantially free of A" means that the content of A is 0.1 mol% or less.

A second bottom product 8 containing the extraction solvent 3 as a main component is obtained from a column bottom side of the solvent recovery column 6. In this manner, the extraction solvent 3 is recovered, and the recovered extraction solvent 3 is reused. The extraction solvent 3 to be reused is heated or cooled by a heat exchanger 9 as necessary, and then supplied to the extraction distillation column 2.

Note that, in Fig. 1, reference numeral 10 denotes a condenser, and reference numeral 11 denotes a heater.

A position (stage) at which the extraction solvent 3 is supplied in the extraction distillation column 2 is preferably a stage located above the stage at which the first mixture 1 is supplied, and the extraction solvent 3 may be supplied to the same stage as the stage at which the reflux is supplied. Optionally, the extraction solvent 3 may be supplied to the same stage as the first mixture 1. Furthermore, the first mixture 1 may be supplied after being mixed with the extraction solvent 3 in advance before being supplied to the extraction distillation column 2.

By the above apparatus and operation, it is possible to separate R22 and HFP from the first mixture 1 containing R22 and HFP and to obtain HFP substantially free of R22.

Note that, the flow of the substance in a case in which the first mixture contains CTFE is as follows.

Specifically, the first mixture 1 containing R22, HFP, and CTFE is supplied to a stage below the central part of the extraction distillation column 2, and the extraction solvent 3 containing the CL compound is supplied to a stage above the supply stage of the first mixture 1 in the extraction distillation column 2 to perform distillation. Then, the first distillate 4 containing HFP as the main component is extracted from the column top side of the extraction distillation column 2, and a mixture containing the extraction solvent as a main component and containing R22 and CTFE is extracted as the first bottom product 5 from the column bottom side of the extraction distillation column 2.

Next, the first bottom product 5 is supplied to the solvent recovery column 6 and distilled to obtain the second distillate 7 substantially free of extraction solvent and containing R22 and CTFE from the column top side of the solvent recovery column 6, and the second bottom product 8 containing the extraction solvent 3 as a main component is obtained from the column bottom side of the solvent recovery column 6. The extraction solvent 3 recovered as the second bottom product 8 is supplied to the extraction distillation column 2 to be reused.

By the above apparatus and operation, it is possible to separate R22 and CTFE from HFP in the first mixture 1 containing R22, CTFE, and HFP and to obtain HFP substantially free of R22 and CTFE.

### [Example]

Hereinafter, an embodiment of the present disclosure will be described in detail with reference to an example, but the embodiment of the present disclosure is not limited thereto.

### [Measurement of Relative Volatility Rv]

A value of relative volatility Rv of R22 to HFP when a CL compound was added to a mixture of R22 and HFP having a molar ratio (HFP/R22) of 10/90 was measured by the above-described method. A relationship between a molar ratio of an added amount of the CL compound to a total amount of the R22 and the HFP (CL/(R22+HFP)) and the relative volatility Rv after adding the CL compound is illustrated in Table 3.

Boiling points of the CL compounds are also illustrated in Table 3.

**[Table 3]**

| CL compound (compound name) | CL compound boiling point (°C) | Molar ratio (CL/(R22+HFP)) | Relative volatility Rv |
|---|---|---|---|
| Methylene chloride | 40 | 3.00 | 0.49 |
| Methylene chloride | 40 | 4.95 | 0.47 |
| Chloroform | 61 | 2.75 | 0.38 |
| Chloroform | 61 | 6.98 | 0.37 |
| Chloroform | 61 | 2.99 | 0.38 |
| Chloroform | 61 | 1.04 | 0.37 |
| Chloroform | 61 | 0.25 | 0.92 |

### [Distillation Simulation]

Using the results described in the above table obtained by measuring the relative volatility Rv of R22 to HFP, the following extraction distillation and solvent recovery distillation were simulated by a calculation method based on known thermodynamic characteristics (Chemcad) (chemical engineering process simulator of Chemstations). The results are illustrated in Table 4. In the table, "-" means that no solvent is used or distillation using a solvent recovery column is not performed.

### (Example 1)

From a 50th stage from a column top of a distillation column with 60 stages, a mixture of R22 and HFP having a molar ratio (HFP/R22) of 15/85 was continuously supplied at 1 kmol per hour to perform distillation, a first distillate was continuously extracted at 0.55 kmol per hour from a column top side, and a first bottom product was continuously extracted at 0.45 kmol per hour from a column bottom side. A pressure in the distillation column during this period was set to 0.5 MPaG (gauge pressure), column top temperature was set to 6.23°C, and column bottom temperature was set to 7.03°C.

A content of each component relative to an entire first distillate was 94.4 mol% for R22 and 5.6 mol% for HFP. A content of each component relative to an entire first bottom product was 73.6 mol% for R22 and 26.4 mol% for HFP.

### (Example 2)

A mixture of R22 and HFP having a molar ratio (HFP/R22) of 15/85 was continuously supplied at a rate of 1 kmol per hour from a 50th stage from a column top of an extraction distillation column having 60 stages, and chloroform was continuously supplied at a rate of 3 kmol per hour from a 15th stage from a column top.

Then, a pressure in the extraction distillation column was set to 0.5 MPaG (gauge pressure), column top temperature was set to 22.09°C, column bottom temperature was set to 57.99°C, and extraction distillation was continuously performed. The first distillate was distilled from the column top side of the extraction distillation column at a rate of 0.12 kmol per hour, and the first bottom product was withdrawn from the column bottom side at a rate of 3.88 kmol per hour.

Chloroform was not detected in an extracted first distillate, a content of HFP relative to an entire first distillate was 99.9 mol%, and a remaining component was R22. In contrast, a content of each component relative to an entire first bottom product was 21.9 mol% for R22 and 0.9 mol% for HFP, and a remaining part was occupied by chloroform as an extraction solvent.

Next, the first bottom product was continuously supplied at 1 kmol per hour from a 10th stage from a column top of a solvent recovery column having 30 stages, and distillation was continuously performed at a pressure in the solvent recovery column of 0.4 MPaG (gauge pressure), column top temperature of 1.0°C, and column bottom temperature of 120.63°C. A second distillate was distilled from a column top side of the solvent recovery column at a rate of 0.88 kmol per hour, and a second bottom product was extracted from a column bottom side at a rate of 3 kmol per hour.

When the composition of each of the second distillate and the second bottom product extracted was analyzed, a content of each component relative to an entire second distillate was 96.1 mol% for R22, 3.9 mol% for HFP, and a remaining part was an extraction solvent. In contrast, a content of each component relative to an entire second bottom product was 99.99 mol% or more for chloroform.

**[Table 4]**

| | | | Example 1 | Example 2 |
|---|---|---|---|---|
| Solvent type | | | - | Chloroform |
| | R22 supply amount | kmol/h | 0.85 | 0.85 |
| | HFP supply amount | kmol/h | 0.15 | 0.15 |
| | Solvent supply amount | kmol/h | - | 3.00 |
| | Column pressure | MPaG | 0.50 | 0.50 |
| | Number of stages | | 60 | 60 |
| | R22 + HFP supply stage | | 50 | 50 |
| | Solvent supply stage | | - | 15 |
| Distillation column or extraction distillation column | Reflux ratio | | 30 | 30 |
| | Column top temperature | °C | 6.23 | 22.09 |
| | R22 distillation amount | kmol/h | 0.52 | 0.00 |
| | HFP distillation amount | kmol/h | 0.03 | 0.12 |
| | Solvent distillation amount | kmol/h | - | 0.00 |
| | HFP purity | Mol% | 5 | 99.9 |
| | Column bottom temperature | °C | 7.03 | 57.99 |
| | R22 bottom product amount | kmol/h | 0.33 | 0.85 |
| | HFP bottom product amount | kmol/h | 0.12 | 0.03 |
| | Solvent bottom product amount | kmol/h | - | 3.00 |
| | Column pressure | MPaG | - | 0.40 |
| | Number of stages | | - | 30 |
| | Supply stage | | - | 10 |
| | Reflux ratio | | - | 15 |
| | Column top temperature | °C | - | 1.00 |
| Solvent recovery column | R22 distillation amount | kmol/h | - | 0.85 |
| | HFP distillation amount | kmol/h | - | 0.03 |
| | Solvent distillation amount | kmol/h | - | 0.00 |
| | Column bottom temperature | °C | - | 120.63 |
| | R22 bottom product amount | kmol/h | - | 0.00 |
| | HFP bottom product amount | kmol/h | - | 0.00 |
| | Solvent bottom product amount | kmol/h | - | 3.00 |

In the above example, Example 2 is a working example, and Example 1 is a comparative example. As illustrated in Table 4, in Example 2, as compared with Example 1, R22 and HFP are separated with high efficiency, and high-purity HFP is obtained.

### INDUSTRIAL APPLICABILITY

According to one embodiment of the present disclosure, R22 and HFP can be efficiently separated from a mixture containing R22 and HFP. According to one embodiment of the present disclosure, for example, HFP useful as a raw material for fluororesin production or the like can be obtained at a high concentration, and there is a great economic advantage.

The disclosure of Japanese Patent Application No. 2022-188720 filed on November 25, 2022 is incorporated herein by reference in its entirety. In addition, all documents, patent applications, and technical standards described in this specification are incorporated herein by reference to the same extent as if each document, patent application, and technical standard were specifically and individually described to be incorporated by reference.

### REFERENCE SIGNS LIST

- 1: First mixture
- 2: Extraction distillation column
- 3: Extraction solvent
- 4: First distillate
- 5: First bottom product
- 6: Solvent recovery column
- 7: Second distillate
- 8: Second bottom product
- 9: Heat exchanger
- 10: Condenser
- 11: Heater

## Claims

1. A method for separating chlorodifluoromethane and hexafluoropropylene, the method comprising:
a mixing step of obtaining a mixture for extraction, which is a mixture of a first mixture comprising chlorodifluoromethane and hexafluoropropylene and an extraction solvent comprising at least one chlorine-containing compound selected from the group consisting of methylene chloride, chloroform, 1,1,1-trichloroethane, 1,1,2-trichloroethane, 1,1,1,2-tetrachloroethane, 1,1,2,2-tetrachloroethane, pentachloroethane, hexachloroethane, trichloroethylene, and tetrachloroethylene; and
an extraction distillation step of distilling the mixture for extraction to respectively obtain a first distillate comprising hexafluoropropylene as a main component, and a first bottom product comprising the extraction solvent as a main component and comprising chlorodifluoromethane.

2. The method for separating chlorodifluoromethane and hexafluoropropylene according to claim 1, wherein the chlorine-containing compound has a boiling point in a range of from 40°C to 130°C.

3. The method for separating chlorodifluoromethane and hexafluoropropylene according to claim 1, wherein the chlorine-containing compound is a compound that reduces a relative volatility Rv of chlorodifluoromethane to hexafluoropropylene to less than 0.9 when the chlorine-containing compound is added in an amount equal to three times a total molar amount of the chlorodifluoromethane and the hexafluoropropylene.

4. The method for separating chlorodifluoromethane and hexafluoropropylene according to claim 1, wherein the chlorine-containing compound is a compound that reduces a relative volatility Rv of chlorodifluoromethane to hexafluoropropylene to less than 0.9 when the chlorine-containing compound is added in an amount equal to a total molar amount of the chlorodifluoromethane and the hexafluoropropylene.

5. The method for separating chlorodifluoromethane and hexafluoropropylene according to claim 1, wherein the extraction solvent comprises at least one chlorine-containing compound selected from the group consisting of 1,1,1-trichloroethane, trichloroethylene, tetrachloroethylene, chloroform, and methylene chloride.

6. The method for separating chlorodifluoromethane and hexafluoropropylene according to claim 1, wherein a molar ratio of the chlorine-containing compound added in the mixing step is from 1/1 to 30/1 relative to a total molar amount of the chlorodifluoromethane and the hexafluoropropylene.

7. The method for separating chlorodifluoromethane and hexafluoropropylene according to any one of claims 1 to 6, further comprising:
a second distillation step of distilling the first bottom product to obtain a second distillate comprising chlorodifluoromethane as a main component.

8. The method for separating chlorodifluoromethane and hexafluoropropylene according to claim 1, wherein:
the first mixture further comprises chlorotrifluoroethylene, and
the first bottom product further comprises chlorotrifluoroethylene.

9. The method for separating chlorodifluoromethane and hexafluoropropylene according to claim 7, wherein:
the first mixture further comprises chlorotrifluoroethylene,
the first bottom product further comprises chlorotrifluoroethylene, and
the second distillate further comprises chlorotrifluoroethylene.
